# EUROPÄISCHE PATENTSCHRIFT

(11) **EP 2 988 049 B1**
(45) Veröffentlichungstag und Bekanntmachung des Hinweises auf die Patenterteilung: **24.04.2019**
(21) Anmeldenummer: 15184840.5
(22) Anmeldetag: 08.07.2009
(51) Int. Cl.: B29C 65/00, B29C 65/36, A61L 27/18, A61L 31/06, A61M 25/00, A61M 25/10, A61M 39/14, A61L 26/00, F16L 31/00, B29C 65/10, B29C 65/14, A61L 29/06, A61M 39/10, A61L 24/04, F16L 47/00, B29C 57/02, B29L 31/00, B29C 65/68, B29C 65/56, B29K 705/12

(54) **VERFAHREN ZUM VERBINDEN VON KUNSTSTOFFRÖHRCHEN**
METHOD FOR CONNECTING PLASTIC TUBES
PROCÉDÉ DE RACCORDEMENT DE PETITS TUBES MÉTALLIQUES

(30) Priorität: 16.07.2008 CH 11062008
(43) Veröffentlichungstag der Anmeldung: 24.02.2016
(62) Teilanmeldung aus: 09797338.2
(73) Patentinhaber: SIS Medical AG, 8500 Frauenfeld (CH)
(72) Erfinder: Schwager, Michael, 8404 Winterthur (CH)
(74) Vertreter: Keller & Partner Patentanwälte AG

(56) Entgegenhaltungen:
- EP-A- 0 774 611
- WO-A-99/22170
- WO-A-2007/140635
- DE-U1- 20 004 312
- FR-A- 2 252 191
- US-A- 3 315 986
- US-A- 5 092 632
- US-A- 5 549 949

## Beschreibung

### Technisches Gebiet

Die Erfindung betrifft ein Verfahren zum koaxialen Verbinden eines ersten Kunststoffröhrchens mit einem zweiten Kunststoffröhrchen, welche Kunststoffröhrchen insbesondere als Bestandteile eines medizinischen Katheters vorgesehen sind, wobei die beiden Kunststoffröhrchen über ein rohrförmiges Verbindungsstück verbunden werden. Des Weiteren bezieht sich die Erfindung auf eine Verwendung des Verfahrens bei der Herstellung eines Katheters und auf eine Anordnung aus zwei miteinander verbundenen Kunststoffröhrchen.

### Stand der Technik

Insbesondere bei der Herstellung von medizinischen Kathetern ist es notwendig, Kunststoffröhrchen mit unterschiedlichen Materialeigenschaften miteinander zu verbinden, um verschiedene Bereiche des Katheters optimal an die jeweiligen Erfordernisse anzupassen. Um eine gute Einführbarkeit eines Katheters zu gewährleisten, wird der Spitzenbereich des Katheters im Allgemeinen aus einem relativ flexiblen Kunststoffröhrchen ausgebildet, während die dahinter liegenden Abschnitte des Katheters aus steiferen Kunststoffröhrchen bestehen.

Die verschiedenartigen Kunststoffröhrchen werden dabei meist miteinander verklebt und/oder miteinander verschweisst.

Ein Verschweissen von Kunststoffröhrchen ist aber im Allgemeinen nur möglich, wenn die zu verbindenden Kunststoffröhrchen bzw. deren Polymermaterialien bezüglich der chemischen Struktur sehr ähnlich sind. In der US 4,563,181 ist beispielsweise eine Stossverschweissung eines Katheterschafts aus Nylon (Polyamid) mit einer Katheterspitze aus einem relativ weichen und mit Nylon kompatiblen Polyether-Polyamid-Copolymer beschrieben. Ein derartiges Verfahren basiert jedoch auf kompatiblen Materialkombinationen, welche meist in aufwändigen Entwicklungsprozessen ermittelt werden müssen.

Beim Verkleben von Kunststoffröhrchen besteht zwar eine grössere Flexibilität, das präzise Aufbringen des Klebstoffs, insbesondere bei den geringen Durchmessern der für Katheter geeigneten Kunststoffröhrchen, ist jedoch ebenfalls aufwändig.

Es besteht daher, insbesondere für die Herstellung von medizinischen Kathetern, nach wie vor Bedarf nach einem Verfahren, welches eine einfachere und sichere Verbindung von Kunststoffröhrchen auch aus chemisch unterschiedlichen Materialien ermöglicht.

### Darstellung der Erfindung

Aufgabe der Erfindung ist es daher, ein dem eingangs genannten technischen Gebiet zugehörendes Verfahren bereit zu stellen, welches flexibler anwendbar ist und eine einfache und gleichzeitig sichere Verbindung von Kunststoffröhrchen, insbesondere bei der Herstellung von medizinischen Kathetern, ermöglicht.

Die Lösung der Aufgabe ist durch die Merkmale des Anspruchs 1 definiert. Gemäss der Erfindung werden das erste Kunststoffröhrchen und/oder das zweite Kunststoffröhrchen in einem Umformprozess von aussen an das rohrförmige Verbindungsstück angeformt, so dass eine adhesive und/oder formschlüssige Verbindung ausgebildet wird.

Unter einem Kunststoffröhrchen wird in diesem Rahmen ein dünnes Rohr aus einem Kunststoff verstanden, welches bevorzugt einen Durchmesser von höchstens 3 mm, besonders bevorzugt einen Durchmesser von höchstens 2 mm, und insbesondere bevorzugt einen Durchmesser von höchstens 1 mm aufweisst.

Eine Wandstärke der Kunststoffröhrchen misst insbesondere höchstens 0.5 mm, besonders bevorzugt höchstens 0.2 mm, und insbesondere bevorzugt höchstens 0.1 mm.

Der Begriff Umformprozess bzw. Umformen bezieht sich in diesem Zusammenhang auf einen Prozess, bei welchem ein Körper, z. B. ein Kunststoffröhrchen, gezielt plastisch verformt und in eine neue Form gebracht wird, wobei das Volumen und die Masse des umgeformten Körpers erhalten beleibt.

Grundsätzlich wird wenigstens eines der beiden zu verbindenden Kunststoffröhrchen durch den erfindungsgemässen Umformprozess mit dem rohrförmigen Verbindungsstück verbunden. Es liegt durchaus im Rahmen der Erfindung, dass das andere Kunststoffröhrchen, falls dies zweckmässig ist, durch eine andere Verbindungstechnik, z. B. durch Stoffschluss, mit dem rohrförmigen Verbindungsstück verbunden wird. Auch möglich ist die Verwendung eines ersten Kunststoffröhrchens, welches an einem Ende bereits über ein rohrförmiges Verbindungsstück verfügt oder die Ausbildung eines rohrförmigen Verbindungsstücks in einem Endbereich des ersten Kunststoffröhrchens.

Während dem Umformprozess findet eine eigentliche plastische Verformung des ersten und/oder des zweiten Kunststoffröhrchen ein. Durch den Umformprozess werden die Grenzflächenschichten der inneren Bereiche des ersten und/oder des zweiten Kunststoffröhrchens in direkten Kontakt mit der Grenzflächenschicht des äusseren Bereichs des rohrförmigen Verbindungsstücks gebracht. Da während dem Umformprozess die Kunststoffröhrchen an das rohrförmige Verbindungsstück bzw. an dessen äussere Kontur angeformt werden, werden die Kontaktflächen der Grenzflächenschichten der Kunststoffröhrchen und der Grenzflächenschichten des rohrförmigen Verbindungsstücks maximiert. Dadurch wird eine adhesive Verbindung bzw. ein mechanischer Zusammenhalt zwischen den Kunststoffröhrchen und dem rohrförmigen Verbindungsstück erhalten, welcher insbesondere durch Verklammerungen auf mikroskopischer Skala und/oder atomare und/oder molekulare Wechselwirkungen zwischen den Grenzflächenschichten der Kunststoffröhrchen und des rohrförmigen Verbindungsstücks beruht.

Derartige adhesive Verbindungen sind insbesondere auch fluiddicht, womit auf eine Abdichtung der Verbindung durch zusätzliche Dichtmassen, z. B. Klebstoffe, verzichtet werden kann. Dies ist insbesondere bei medizinischen Kathetern von Vorteil.

Je nach Ausgestaltung des äusseren Bereichs des rohrförmigen Verbindungsstücks kann während dem Umformprozess auch eine formschlüssige Verbindung auf makroskopischer Skala gebildet, welche insbesondere zusätzlich zur adhesiven Verbindung die mechanische Stabilität der Verbindung zwischen den Kunststoffröhrchen und dem rohrförmigen Verbindungsstück erhöht.

Durch den erfindungsgemässen Umformprozess lassen sich klebstofffreie und mechanisch stabile Verbindungen zwischen den Kunststoffröhrchen und dem rohrförmigen Verbindungsstück bilden. Dies ist insbesondere bei Kunststoffröhrchen, welche einen Durchmesser von höchstens 3 mm aufweisen, von grossem Vorteil. So ist z. B. das gleichmässige und präzise Anbringen von Klebstoff bei derartig dünnen Kunststoffröhrchen in der Praxis relativ aufwändig. Zudem besteht die Gefahr, dass beim Anbringen des Klebstoffs dieser versehentlich ins Innere des Kunststoffröhrchens gelangt. Das Entfernen des ungewollt in das Kunststoffröhrchen eingebrachten Klebstoffs ist dann entsprechend schwierig und mühsam. Entsprechend müssen auch Schweissvorgänge an derartig dünnen Kunststoffröhrchen unter anderem aufgrund der geringen Wandstärken sehr präzise durchgeführt werden, damit die Kunststoffröhrchen nicht beschädigt werden. Zudem stellt das Verschweissen von Kunststoff gewisse Anforderungen an die verwendeten Materialien, da sich nicht alle Kunststoffe untereinander verschweissen lassen. Das Verschweissen von Kunststoff mit anderen Materialien, wie z. B. Metall, ist zudem nicht möglich, was die Materialwahl für das rohrförmige Verbindungsstück, welches nicht zwingend aus einem Kunststoff bestehen muss, einschränkt.

Da die Kunststoffröhrchen von aussen an das rohrförmige Verbindungselement angeformt werden, kann das rohrförmige Verbindungselement insbesondere vollständig von den Kunststoffröhrchen umhüllt bzw. umfasst werden. Damit werden allfällige Kanten und/oder Vorstände des rohrförmigen Verbindungsstück von den Kunststoffröhrchen abgedeckt. Kantenbereiche der Kunststoffröhrchen selbst können z. B. während dem erfindungsgemässen Umformprozess insbesondere abgerundet und/oder keilförmig auslaufend ausgebildet werden. Dies kann z. B. bei Kathetern von grossem Vorteil sein, da diese durch empfindliche und teilweise enge Hohlorgane des menschlichen und/oder tierischen Körpers geführt werden müssen. Vorstände oder Kanten an der Verbindungsstelle würden die Hohlorgane beschädigen bzw. verletzen.

Es hat sich gezeigt, dass die erfindungsgemäss herstellbaren Verbindungen eine hohe mechanische Stabilität aufweisen und insbesondere auch gegenüber Zugbelastungen äusserst stabil sind.

Somit bietet das erfindungsgemässe Verfahren, insbesondere gegenüber dem Verschweissen und/oder Verkleben von Kunststoffröhrchen, wesentliche Vorteile und ist zudem auch bei unterschiedlichen Materialien flexibel einsetzbar. Da das erfindungsgemässe Verfahren speziell bei sehr dünnen Kunststoffröhrchen vorteilhaft ist, eignet es sich im Besonderen für die Herstellung von medizinischen Kathetern.

Bevorzugt werden das erste Kunststoffröhrchen und/oder das zweite Kunststoffröhrchen während dem Umformprozess durch eine Wärmebehandlung erweicht und durch eine in einer radialen Richtung einwirkende Presskraft an das rohrförmige Verbindungsstück angeformt. Durch die Wärmebehandlung wird die Grenzschicht des ersten und/oder des zweiten Kunststoffröhrchens erweicht, wodurch sich die Anformung an das rohrförmige Verbindungsstück vereinfacht. Die Presskraft, welche insbesondere während der Wärmebehandlung auf das erste und/oder das zweite Kunststoffröhrchen einwirkt, kann dieses bestmöglich an die äussere Kontur des rohrförmigen Verbindungsstücks angeformt werden, da das Kunststoffröhrchen in diesem Fall in erweichtem Zustand vorliegt. Damit bildet sich eine grösstmögliche Adhäsion zwischen den Kunststoffröhrchen und dem rohrförmigen Verbindungsstück aus.

Die Presskraft wirkt mit Vorteil in sämtliche radiale Richtungen auf das Kunststoffröhrchen ein, so dass das Kunststoffröhrchen aus allen Richtungen gleichmässig an das rohrförmige Verbindungsstück angepresst wird.

Zur Durchführung des Umformprozesses ist insbesondere ein Schrumpfschlauch geeignet, welcher z. B. aus einem Kunststoff wie beispielsweise Polyolefine, Polyvinylchlorid oder Teflon besteht. Der Schrumpfschlauch kann direkt über die Bereiche der Kunststoffröhrchen geschoben werden, welche im Umformprozess an das rohrförmige Verbindungsstück angeformt werden sollen. Durch Zufuhr von Wärme, beispielsweise in Form von Heissluft und/oder elektromagnetischer Strahlung, kann der Schrumpfschlauch erhitzt werden, so dass er sind in radialer Richtung stark zusammenzieht und eine Presskraft auf die Kunststoffröhrchen einwirkt. Durch die zugeführte Wärme können gleichzeitig die Kunststoffröhrchen erweicht und/oder angeschmolzen werden. Der Schrumpfschlauch kann nach dem Umformprozess wieder entfernt werden oder aber auf den verbundenen Kunststoffröhrchen belassen werden. In letzterem Fall kann der Schrumpfschlauch als zusätzliche Stabilisierung und/oder als Schutz vor die Verbindungsstelle dienen.

Grundsätzlich liegt es aber auch im Rahmen der Erfindung, zum Umformen während dem Umformprozess lediglich eine Presskraft einwirken zu lassen, falls dies aufgrund der Materialeigenschaften des Kunststoffröhrchens möglich ist. Dies geht jedoch zu Lasten einer bestmöglichen Adhäsion zwischen Kunststoffröhrchen und rohrförmigem Verbindungsstück.

Insbesondere wird vor dem Umformprozess ein Endbereich des ersten Kunststoffröhrchens koaxial über ein erstes Ende des rohrförmigen Verbindungsstücks geschoben und ein Endbereich des zweiten Kunststoffröhrchens wird koaxial über ein zweites Ende des rohrförmigen Verbindungsstücks geschoben. Das rohrförmige Verbindungsstück liegt dabei z. B. als separater Hohlzylinder bzw. als kurzes Rohrstück vor. Damit können die beiden zu verbindenden Kunststoffröhrchen vor dem Umformprozess bereits in ihrer vorgesehenen Lage angeordnet werden. Anschliessend kann der Umformprozess z. B. für beide Kunststoffröhrchen gleichzeitig durchgeführt werden, was insbesondere Zeit spart.

Es ist aber auch denkbar, in einem ersten Schritt den Endbereich des ersten Kunststoffröhrchens mit dem ersten Ende des rohrförmigen Verbindungsstücks zu verbinden und erst danach den Endbereich des zweiten Kunststoffröhrchens mit dem zweiten Ende des rohrförmigen Verbindungsstücks zu verbinden.

Grundsätzlich ist es auch möglich, ein erstes Kunststoffröhrchen, welches mit einem weiteren und aus dem ersten Kunststoffröhrchen herausragenden Innenröhrchen bestückt ist, mit dem zweiten Kunststoffröhrchen zu verbinden. Hierfür weist das rohrförmigen Verbindungsstück insbesondere einen Innendurchmesser auf, welcher wenigstens gleich gross ist wie der Aussendurchmesser des Innenröhrchens des ersten Kunststoffröhrchens. Das rohrförmige Verbindungsstück kann in diesem Fall auf das Innenröhrchen aufgeschoben werden und anschliessend kann das erste Ende des rohrförmigen Verbindungsstücks in den Endbereich des ersten Kunststoffröhrchens eingeführt werden. Danach kann das zweite Kunststoffröhrchen zuerst über das aus dem ersten Kunststoffröhrchen herausragende Innenröhrchen und dann über das zweite Ende des rohrförmigen Verbindungsstücks geschoben werden.

Bevorzugt ist ein Elastizitätsmodul des rohrförmigen Verbindungsstücks grösser als ein Elastizitätsmodul des ersten Kunststoffröhrchens und grösser als ein Elastizitätsmodul des zweiten Kunststoffröhrchens. Eine derartige Anordnung ist insbesondere gegenüber Zugbelastungen stabiler. Unter Zugbelastung haben die Kunststoffröhrchen nämlich die Tendenz, sich im Durchmesser zusammen zu ziehen. Dies erzeugt eine Kraft in radialer Richtung, welche auf das rohrförmige Verbindungsstück einwirkt. Je grösser das Elastizitätsmodul des rohrförmigen Verbindungsstücks, desto besser kann dieses der in radialer Richtung einwirkenden Kraft entgegen halten, ohne selber stark verformt zu werden. Damit wird unter Zugbelastung zwischen den Kunststoffröhrchen und dem rohrförmigen Verbindungsstück zusätzlich ein Kraftschluss erzeugt, welcher die mechanische Festigkeit der Verbindung steigert.

Grundsätzlich ist es auch möglich, ein rohrförmiges Verbindungsstück einem geringerem Elastizitätsmodul vorzusehen. Dies kann unter Umständen vorteilhaft sein, um die Flexibilität der Verbindungsstelle zu erhöhen. Allerdings entfällt in diesem Fall der Vorteil des zusätzlichen Kraftschlusses zwischen rohrförmigen Verbindungsstück und Kunststoffröhrchen.

Bevorzugt besteht das verwendete erste Kunststoffröhrchen aus einem ersten thermoplastischen Material, beispielsweise aus Polyethylentherephtalat, und das zweite Kunststoffröhrchen aus einem zweiten thermoplastischen Material, beispielsweise aus Nylon. Derartige thermoplastische Materialien lassen sich durch Wärmebehandlung problemlos erweichen bzw. anschmelzen und in erweichtem Zustand umformen. Da das Erweichen bzw. Anschmelzen des thermoplastischen Materials reversibel ist, werden die Materialeigenschaften der Kunststoffröhrchen durch den Umformprozess nicht verändert und/oder beeinträchtigt.

Anordnungen aus Kunststoffröhrchen aus derartigen thermoplastischen Materialen sind insbesondere für medizinische Katheter geeignet. Dabei kann beispielsweise ein Kunststoffröhrchen aus Polyethylentherephtalat als Katheterschaft und ein Kunststoffröhrchen aus Nylon im Bereich der Katheterspitze angeordnet sein. Da Kunststoffröhrchen aus Polyethylenetherephtalat bei gleichen Dimensionen im Allgemeinen steifer sind als Kunststoffröhrchen aus Nylon, lassen sich derartig ausgebildete Katheter gut in Hohlorgane einführen.

Grundsätzlich ist es aber auch denkbar, Kunststoffröhrchen aus anderen umformbaren Kunststoffen einzusetzen.

Es kann auch vorteilhaft sein, den Endbereich des ersten Kunststoffröhrchens vor dem Umformprozess zusätzlich koaxial über den Endbereich das zweiten Kunststoffröhrchen zu schieben. Der Endbereich des ersten Kunststoffröhrchens wird in diesem Fall während dem Umformprozess wenigstens teilweise an den Endbereich des zweiten Kunststoffröhrchens angeformt. Damit kann insbesondere verhindert werden, dass zwischen den beiden zu verbindenden Kunststoffröhrchen ein Spalt gebildet wird, wodurch die Dichtigkeit der Verbindung zwischen den beiden Kunststoffröhrchen verbessert wird. Es ist aber auch möglich, die beiden zu verbindenden Kunststoffröhrchen stirnseitig aneinander zu schieben. Die Verbindung zwischen den beiden Kunststoffröhrchen ist dann zwar unter Umständen weniger dicht, es ergibt sich aber der Vorteil, dass der Durchmesser der Verbindungsstelle insgesamt dünner ausfällt.

Bevorzugt ist dabei ein Elastizitätsmodul des ersten Kunststoffröhrchens grösser als ein Elastizitätsmodul des zweiten Kunststoffröhrchens. Dies verbessert insbesondere die mechanische Festigkeit der direkten Verbindung zwischen den beiden Kunststoffröhrchen. Da sich das erste Kunststoffröhrchen, welches innerhalb des Endbereichs des zweiten Kunststoffröhrchen angeordnet ist, unter Zugbelastung weniger stark zusammenzieht als das darüber liegende zweite Kunststoffröhrchen, wird unter Zugbelastung zusätzlich eine kraftschlüssige Verbindung direkt zwischen dem ersten Kunststoffröhrchen und dem zweiten Kunststoffröhrchen erzeugt.

Es ist aber auch möglich, ein erstes Kunststoffröhrchen mit einem Elastizitätsmodul einzusetzen, welches geringer ist als das Elastizitätsmodul des zweiten Kunststoffröhrchens. In diesem Fall entfällt jedoch die Verbesserung der mechanischen Festigkeit unter Zugbelastung.

Insbesondere besteht das verwendete rohrförmige Verbindungsstück aus Polyimid. Polyimid als Material für das rohrförmige Verbindungsstück hat sich als vorteilhaft erwiesen, da Polyimid ein relativ grosses Elastizitätsmodul aufweist und damit schwer deformierbar ist. Gleichzeitig ist aber eine gewisse Flexibilität vorhanden, welche insbesondere bei der Verbindung von flexiblen Kunststoffröhrchen wünschenswert ist. Des Weiteren ist die Schrumpfung von Polyimid unter Wärmeeinwirkung im hier interessierenden Temperaturbereich äusserst gering. Ein rohrförmiges Verbindungsstück auch Polyimid bleibt daher auch bei den während dem Umformprozess herrschenden Temperaturen äusserst formstabil.

Grundsätzlich kann anstelle eines rohrförmigen Verbindungsstücks aus Polyimid auch ein Verbindungsstück aus Stahl oder sonst einem schwer deformierbaren Material verwendet werden, welches unter Wärmeeinwirkung möglichst wenig schrumpft.

Insbesondere ist eine Wandung des rohrförmigen Verbindungsstücks mit einer eingebetteten Drahtwendel verstärkt. Während dem Umformprozess kann die Wandung des rohrförmigen Verbindungsstücks, welches z. B. aus einem thermoplastischen Material besteht, an die eingebettete Drahtwendel angeformt werden. Dadurch wird eine schraubenlinienförmige Struktur an der Aussenseite des rohrförmigen Verbindungsstücks gebildet. Das erste und/oder das zweite Kunststoffröhrchen werden in diesem Fall während dem Umformprozess an die schraubenlinienförmige Struktur angeformt, womit eine formschlüssige Verbindung zwischen den Kunststoffröhrchen und dem rohrförmigen Verbindungsstück ausgebildet wird. Dies verbessert die mechanische Festigkeit der Verbindung bzw. der Anordnung der Kunststoffröhrchen und des rohrförmigen Verbindungsstücks.

Es ist aber auch möglich, auf eine eingebettete Drahtwendel zu verzichten und/oder rohrförmige Verbindungsstücke mit strukturierten Aussenseiten zu verwenden.

In einer bevorzugten Variante weist daher das rohrförmige Verbindungsstück eine strukturierte äussere Mantelfläche auf. Die strukturierte äussere Mantelfläche kann beispielsweise Vorstände und/oder Rillen und/oder Schraubengewinde und/oder umlaufende Flansche umfassen. Da die Kunststoffröhrchen während dem Umformprozess an die strukturierte äussere Mantelfläche des rohrförmigen Verbindungsstücks angeformt werden, wird eine formschlüssige Verbindung zwischen Kunststoffröhrchen und rohrförmigem Verbindungsstück gebildet, welche die mechanische Festigkeit verbessert.

Es ist aber auch möglich, rohrförmige Verbindungsstücke ohne strukturierte Aussenseiten bzw. Mantelflächen einzusetzen. Allerdings ist dabei die mechanische Festigkeit der Verbindung gegenüber einer strukturierten äusseren Mantelfläche vermindert.

In einer weiteren vorteilhaften Variante wird als rohrförmiges Verbindungsstück eine Schraubenfeder verwendet, wobei die Schraubenfeder insbesondere aus metallischem Draht besteht. Schraubenfedern als rohrförmige Verbindungsstücke haben sich als vorteilhaft erwiesen, da sie zwar eine relativ hohe Biegeflexibilität bezüglich der Längsachse aufweisen, gleichzeitig aber in radialer Richtung nur wenig flexibel sind. Zudem weisen Schraubenfedern aufgrund der nebeneinander angeordneten Drahtwindungen eine strukturiert äussere Mantelfläche auf. Da die Schraubenfeder in radialer Richtung nur wenig flexibel ist, lassen sich die Kunststoffröhrchen im Umformprozess gut an die Schraubenfedern bzw. die einzelnen Drahtwindungen anformen. Dadurch kann in einfacher Art und Weise eine formschlüssige Verbindung zwischen Schraubenfeder und Kunststoffröhrchen hergestellt werden. Derartig hergestellte Anordnungen aus Kunststoffröhrchen, welche über eine Schraubenfeder verbunden sind, zeichnen sich durch eine hohe mechanische Festigkeit aus, sind aber bezüglich einer Längsrichtung dennoch relativ flexibel.

Bevorzugt weisen einzelne Windungen der Schraubenfeder einen Abstand auf, welcher 10 - 50% eines Drahtdurchmessers der Schraubenfeder entspricht. Eine Anordnung aus Kunststoffröhrchen, welche über eine Schraubenfeder mit derartig beabstandeten einzelnen Windungen verbunden sind, zeichnet sich insbesondere durch eine optimale Flexibilität im Bereich der Verbindungsstelle aus. Insbesondere die Biegeflexibilität der Verbindungsstelle bezüglich einer Längsachse ist damit optimiert.

Es ist aber grundsätzlich auch möglich, eine Schraubenfeder einzusetzen, bei welcher die einzelnen Drahtwindungen einen Abstand von mehr als 50% des Drahtdurchmessers der Schraubenfeder aufweisen. In diesem Fall besteht jedoch die Gefahr, dass während dem Umformprozess die Kunststoffröhrchen in den Innenbereich der Schraubenfeder hinein gedrückt werden. Zudem wird die Biegeflexibilität bezüglich der Längsachse durch den grösseren Abstand nicht mehr wesentlich verbessert. Auch geringere Abstände von weniger als 10% des Drahtdurchmessers sind prinzipiell möglich. Allerdings nimmt dann die Biegeflexibilität entsprechend ab.

Es ist aber auch möglich, einzelne Windungen der Schraubenfeder aneinander anliegend anzuordnen. Dies kann vorteilhaft sein, wenn beispielsweise die innere Mantelfläche der Schraubenfeder möglichst gleichmässig ausgebildet sein soll. Insbesondere, falls die hergestellte Anordnung aus den mit der Schraubenfeder verbundenen Kunststoffröhrchen Bestandteil eines Führungsdrahtlumens eines medizinischen Katheters ist, kann dies vorteilhaft sein, da einzeln vorstehende und die Bewegung des Führungsdrahts hindernde Windungen der Schraubenfeder vermieden werden.

Bei Verwendung eines separaten rohrförmigen Verbindungsstücks werden bevorzugt sowohl das erste Kunststoffröhrchen als auch das zweite Kunststoffröhrchen formschlüssig an das rohrförmige Verbindungsstück angeformt. Damit wird eine bezüglich mechanischer Festigkeit optimale Verbindung geschaffen.

Grundsätzlich liegt es aber auch im Rahmen der Erfindung, lediglich das erste Kunststoffröhrchen oder das zweite Kunststoffröhrchen formschlüssig an das rohrförmige Verbindungsstück anzuformen. In diesem Fall kann das nicht formschlüssig verbundene Kunststoffröhrchen beispielsweise adhesiv befestigt werden. Ebenso liegt es im Rahmen der Erfindung, beide Kunststoffröhrchen ohne Formschluss, beispielsweise adhesiv mit dem rohrförmigen Verbindungsstück zu verbinden.

In einer weiteren vorteilhaften Ausführungsform wird vor dem Umformprozess der erste Endbereich des ersten Kunststoffröhrchens zu einem Verbindungsstutzen umgeformt. Das rohrförmige Verbindungsstück besteht in diesem Fall aus einem zu einem Verbindungsstutzen umgeformten Endbereich des ersten Kunststoffröhrchens, wobei der Verbindungsstutzen bevorzugt im Wesentlichen einen geringeren Aussendurchmesser aufweist als das erste Kunststoffröhrchen. Der Verbindungsstutzen übernimmt in diesem Fall die Funktion des rohrförmigen Verbindungsstücks. Damit kann auf ein separates rohrförmiges Verbindungsstück verzichtet werden. Wie vorstehend erwähnt, kann das rohrförmige Verbindungsstück aber auch als separates Teil vorliegen.

Bevorzugt ist dabei ein Elastizitätsmodul des ersten Kunststoffröhrchens grösser als ein Elastizitätsmodul des zweiten Kunststoffröhrchens. Da der Verbindungsstutzen in diesem Fall aus dem ersten Kunststoffröhrchen gebildet wird, weist der Verbindungsstutzen ein Elastizitätsmodul auf, welches grösser ist als das Elastizitätsmodul des zweiten Kunststoffröhrchens. Damit wird eine höhere mechanische Festigkeit der Verbindung zwischen dem Verbindungsstutzen und dem zweiten Kunststoffröhrchen erhalten. Unter Zugbelastung zieht sich das zweite und aussen liegende Kunststoffröhrchen zusammen und drückt in radialer Richtung auf den Verbindungsstutzen. Aufgrund des höheren Elastizitätsmoduls des Verbindungsstutzens zieht sich dieser weniger stark zusammen als das zweite Kunststoffröhrchen. Dies resultiert insgesamt in einer zusätzlichen kraftschlüssigen Verbindung zwischen dem zweiten Kunststoffröhrchen und dem Verbindungsstutzen.

Prinzipiell kann auch das zweite Kunststoffröhrchen ein höheres Elastizitätsmodul aufweisen als das erste Kunststoffröhrchen. Allerdings besteht dabei die Gefahr, dass unter Zugbelastung der Verbindungsstutzen aus dem zweiten Kunststoffröhrchen heraus rutscht, da sich der Verbindungsstutzen unter Zugbelastung in diesem Fall stärker zusammenzieht als das zweite Kunststoffröhrchen.

Bevorzugt wird bei der Umformung des ersten Kunststoffröhrchens zum Verbindungsstutzen ein Aussendurchmesser des Verbindungsstutzens verglichen mit dem Aussendurchmesser des ersten Kunststoffröhrchen im Wesentlichen verjüngt ausgebildet. Damit lässt sich das zweite Kunststoffröhrchen nachher einfacher über den Verbindungsstutzen schieben. Des Weiteren kann so der Aussendurchmesser der Verbindungsstelle minimiert werden, was insbesondere bei medizinischen Kathetern von Vorteil ist.

Es ist aber prinzipiell auch denkbar, den Aussendurchmesser des Verbindungsstutzens verglichen mit dem Aussendurchmesser des ersten Kunststoffröhrchens grösser auszubilden. Dies kann z. B. vorteilhaft sein, um zusätzlich eine formschlüssige Verbindung zwischen den beiden Kunststoffröhrchen zu erzeugen.

Zur Herstellung des Verbindungsstutzens wird bevorzugt ein Endabschnitt eines zylindrischen Drahtstücks in den ersten Endbereich des ersten Kunststoffröhrchens eingeführt und anschliessend der erste Endbereich des ersten Kunststoffröhrchens durch Umformung von aussen an den Endabschnitt des zylindrischen Drahtstücks angeformt. Die Verwendung eines derartigen Drahtstücks ermöglicht insbesondere die Herstellung eines Verbindungsstutzens mit gleichmässiger Wandstärke und klar definiertem Innendurchmesser.

Ein Aussendurchmesser des Endabschnitts des zylindrischen Drahtstücks misst dabei insbesondere 60 - 80%, besonders bevorzugt 65 - 75%, des Innendurchmessers des ersten Kunststoffröhrchens vor der Ausbildung des Verbindungsstutzens.

Es hat sich gezeigt, dass mit derartig dimensionierten Drahtstücken bei den meisten Kunststoffröhrchen sehr gute Ergebnisse bezüglich der Qualität der Verbindungsstutzen erreichbar sind. Insbesondere weisen derartig hergestellte Verbindungsstutzen Stabilitäten auf, welche mit der Stabilität der Kunststoffröhrchen als solche vergleichbar sind. Zudem wird sichergestellt, dass der Verbindungsstutzen eine ausreichende Durchlässigkeit aufweist und gleichzeitig eine signifikante Verjüngung im Vergleich mit dem Kunststoffröhrchen vor der Ausbildung des Verbindungsstutzens erzielbar ist.

Insbesondere wird das zweite Kunststoffröhrchen vor dem Umformprozess koaxial und wenigstens über eine gesamte Länge des Verbindungsstutzens über diesen geschoben. Damit wird sichergestellt, dass während dem nachfolgenden Umformprozess eine maximale Kontaktfläche zwischen dem Verbindungsstutzen und den zweiten Kunststoffröhrchen gebildet wird, wodurch, wie vorstehend bereits erwähnt, die adhesive Verbindung bzw. der mechanische Zusammenhalt optimiert wird.

Es ist aber auch möglich, das zweite Kunststoffröhrchen weiter zu schieben, so dass es über den Verbindungsstutzen hinaus über das erste Kunststoffröhrchen zu liegen kommt. Dies kann insbesondere dann von Vorteil sein, wenn der Verbindungsstutzen einen grösseren Aussendurchmesser aufweist als das erste Kunststoffröhrchen, da dann während dem Umformprozess zusätzlich eine formschlüssige Verbindung herstellbar ist.

Es ist jedoch auch eine Anordnung praktikabel, bei welcher das zweite Kunststoffröhrchen lediglich teilweise über den Verbindungsstutzen geschoben wird. Je kürzer der gemeinsame Kontaktbereich zwischen zweitem Kunststoffröhrchen und dem Verbindungsstutzen ausfällt, desto geringer ist jedoch die adhesive Verbindung.

Besonders bevorzugt wird ein freies Ende des Verbindungsstutzens vor dem Umformprozess aufgeweitet. Indem das freie Ende gegenüber den übrigen Bereichen des Verbindungsstutzens aufgeweitet ausgeformt wird, lässt sich im nachfolgenden Umformprozess eine formschlüssige Verbindung erzeugen, welche insbesondere den mechanischen Zusammenhalt zwischen Verbindungsstutzen und zweitem Kunststoffröhrchen verbessert.

Es ist aber prinzipiell auch möglich, anstelle oder zusätzlich zum freien Ende einen anderen Bereich des Verbindungsstutzens aufzuweiten, welcher z. B. hinter dem freiem Ende des Verbindungsstutzens liegt. Auch möglich ist es, auf eine Aufweitung des Verbindungsstutzens ganz zu verzichten. Allerdings geht dies zu Lasten eines maximalen Zusammenhalts zwischen Verbindungsstutzen und zweitem Kunststoffröhrchen.

Zur Aufweitung des freien Endes des Verbindungsstutzens wird vorteilhaft ein an den Endabschnitt des zylindrischen Drahtstücks anschliessender verdickter Bereich des zylindrischen Drahtstücks und/oder ein aufgeweiteter Übergangsbereich zum verdickten Bereich in einer Richtung zum ersten Kunststoffröhrchen hin in das freie Ende des Verbindungsstutzens hinein geschoben. Der Übergang zwischen dem Endabschnitt des zylindrischen Drahtstücks und dem verdickten Bereich des zylindrischen Drahtstücks ist dabei insbesondere konisch und/oder abgerundet ausgebildet, so dass der verdickte Bereich des zylindrischen Drahtstücks bestmöglich in das freie Ende des Verbindungsstutzens hinein geschoben werden.

Es ist aber beispielsweise auch möglich, für die Aufweitung des Verbindungsstutzens einen konisch zulaufenden Draht einzusetzen und/oder das freie Ende des Verbindungsstücks durch Anschmelzen aufzuweiten.

Bevorzugt entspricht ein Aussendurchmesser des verdickten Bereichs des zylindrischen Drahtstücks im Wesentlichen dem Innendurchmesser des ersten Kunststoffröhrchens vor der Herstellung des Verbindungsstutzens. Es hat sich gezeigt, dass ein derartig dimensioniertes Drahtstück besonders geeignet ist, um eine für eine formschlüssige Verbindung ausreichende Aufweitung des Verbindungsstutzens zu erzielen, ohne den Verbindungsstutzen während der Aufweitung zu beschädigen.

Bevorzugt wird während dem Umformprozess das zweite Kunststoffröhrchen unter Ausbildung einer formschlüssigen Verbindung an den endseitig aufgeweiteten Verbindungsstutzen angeformt. Dadurch wird eine äusserst stabile Verbindung zwischen dem zweiten Kunststoffröhrchen und dem Verbindungsstutzen bzw. dem damit verbundenen ersten Kunststoffröhrchen erhalten.

Mit Vorteil verbleiben während dem Umformprozess der Endabschnitt des zylindrischen Drahtstücks im Verbindungsstutzen und der aufgeweitete Übergangsbereich des zylindrischen Drahtstücks und/oder der verdickte Bereich des zylindrischen Drahtstücks im aufgeweiteten freien Ende des Verbindungsstutzens. Damit kann eine vorgegebene Durchlässigkeit der Verbindungsstelle garantiert werden, da sich der Verbindungsstutzen während dem Umformprozess auch bei Anwendung einer Presskraft und unter Wärmeeinfluss nicht weiter verjüngen kann. Dies ist insbesondere entscheidend, falls die Kunststoffröhrchen beispielsweise zur Fluidleitung und/oder als Führungskanal für einen Draht bei einem Katheter vorgesehen sind. Zudem wird damit garantiert, dass das zweite Kunststoffröhrchen optimal an den Verbindungsstutzen angeformt werden kann, da der Verbindungsstutzen innenseitig gestützt wird. Der verdickte Bereich des zylindrischen Drahtstücks im aufgeweiteten Ende des Verbindungsstutzens, garantiert insbesonders, dass sich der aufgeweitete Bereich während dem Umformprozess nicht wieder verjüngt.

Damit wird die Ausbildung einer formschlüssigen Verbindung ermöglicht, wobei der Verbindungsstutzen an einem freien Ende insbesondere auch nach dem Umformprozess aufgeweitet vorliegt und das zweite Kunststoffröhrchen formschlüssig an den Verbindungsstutzen angeformt ist.

Es ist aber auch denkbar, das zylindrische Drahtstück vor dem Umformprozess aus dem Verbindungsstutzen zu entfernen. Allerdings muss der Umformprozess dann entsprechend sehr genau kontrolliert werden, was unter Umständen aufwändig ist.

In einer weiteren bevorzugten Variante werden das erste Kunststoffröhrchen und/oder das zweite Kunststoffröhrchen nach dem Umformprozess spiralförmig aufgewickelt und unter Anwendung von Wärme zu einer formstabilen Spirale geformt. Das erste Kunststoffröhrchen und/oder das zweite Kunststoffröhrchen liegt daher nach der Anwendung von Wärme in Form einer formstabilen Spirale vor. Damit lassen sich auch längere Kunststoffröhrchen platzsparend, beispielsweise in einer Verpackungshülle, verpacken und lagern. Da die aufgewickelte Spirale nach der Anwendung von Wärme formstabil ist, behält sie auch nach einem Entfernen der Verpackungshülle ihre spiralförmige Form bei. Eine unkontrolliertes Auseinanderrollen des Kunststoffröhrchens wird so wirksam verhindert. Es ist aber auch möglich, auf eine spiralförmige Aufwicklung und/oder die Ausbildung einer formstabilen Spirale zu verzichten.

Insbesondere durch das erfindungsgemässe Verfahren lassen sich demnach Anordnungen herstellen, welche ein erstes Kunststoffröhrchen, das über ein rohrförmiges Verbindungsstück mit einem zweiten Kunststoffröhrchen verbunden ist, umfassen, wobei das erste Kunststoffröhrchen und/oder das zweite Kunststoffröhrchen von aussen an das rohrförmige Verbindungsstück angeformt und adhesiv und/oder formschlüssig mit dem Verbindungsstück verbunden ist.

Derartige Anordnungen können insbesondere durch das erfindungsgemässe Verfahren mit geringen Aussendurchmessern ausgebildet werden. Damit sind die erfindungsgemässen Anordnungen und das erfindungsgemässe Verfahren im Besonderen geeignet zur Verwendung bei der Herstellung von medizinischen Kathetern.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1a: Ein Längsschnitt durch ein erstes Kunststoffröhrchen mit einem daraus herausragenden Innenrohr, wobei ein rohrförmiges Verbindungsstück über das Innenrohr geschoben wurde;
- Fig. 1b: Ein Längsschnitt durch das rohrförmige Verbindungsstück, welches in einen Endbereich eines ersten Kunststoffröhrchens und einen Endbereich eines zweiten Kunststoffröhrchens eingeschoben ist;
- Fig. 1c: Ein Längsschnitt durch die Anordnung aus Fig. 1b, wobei der Endbereich des ersten Kunststoffröhrchens zusätzlich über den Endbereich des zweiten Kunststoffröhrchens geschoben und von einem Schrumpfschlauch umgeben wurde;
- Fig. 1d: Ein Längsschnitt durch die Anordnung aus Fig. 1c nach erfolgtem Umformprozess und Entfernung des Schrumpfschlauchs;
- Fig. 2a: Ein Längsschnitt durch ein in ein erstes Kunststoffröhrchen hinein ragendes zylindrisches Drahtstück;
- Fig. 2b: Ein Längsschnitt durch die Anordnung aus Fig. 2a nach erfolgter Umformung des Endbereichs des ersten Kunststoffröhrchens zu einem verjüngten Verbindungsstutzen;
- Fig. 2c: Ein Längsschnitt durch die Anordnung aus Fig. 2b, wobei zusätzlich ein zweites Kunststoffröhrchen über das zylindrische Drahtstück und den Verbindungsstutzen geschoben wurde;
- Fig. 2d: Ein Längsschnitt durch die Anordnung aus Fig. 2c, nachdem ein freies Ende des Verbindungsstutzens durch einen verdickten Bereich des zylindrischen Drahtstücks aufgeweitet und ein Schrumpfschlauch um die Verbindungsstelle angeordnet wurde;
- Fig. 2e;: Ein Längsschnitt durch die Anordnung aus Fig. 2d nach erfolgtem Umformprozess und Entfernung des Schrumpfschlauchs;
- Fig. 3: Ein Längsschnitt durch eine Variante von Fig. 1d, wobei als rohrförmiges Verbindungsstück eine Schraubenfeder angeordnet ist;
- Fig. 4: Ein zu einer formstabilen Spirale umgeformter medizinischer Katheter, welcher die Anordnung aus Fig. 2e umfasst.

Grundsätzlich sind in den Figuren gleiche Teile mit gleichen Bezugszeichen versehen.

### Wege zur Ausführung der Erfindung

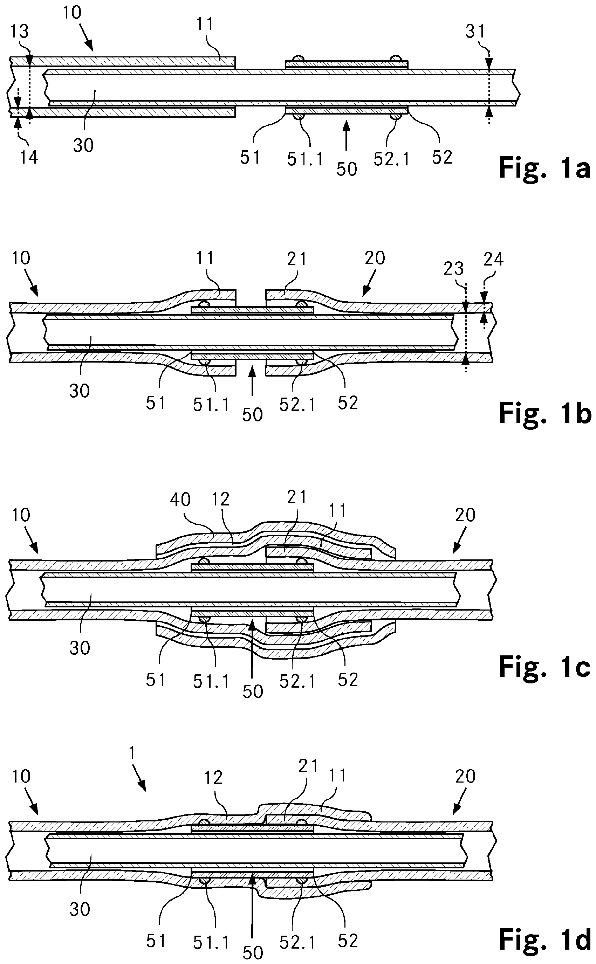

In den Fig. 1a-1c sind verschiedene Zwischenstufen dargestellt, welche bei der Durchführung eines ersten erfindungsgemässen Verfahrens durchlaufen werden. Fig. 1d zeigt eine erste durch das erfindungsgemässe Verfahren herstellbare Anordnung 1.

Auf der linken Seite in Fig. 1a ist ein Endbereich 11 eines ersten Kunststoffröhrchens 10 im Längsschnitt abgebildet. Das erste Kunststoffröhrchen 10 ist zylindrisch und weist einen Innendurchmesser 13 von beispielsweise 0.7 mm bei einer Wandstärke 14 von z. B. 0.1 mm auf und besteht aus Polyethylentherephtalat (PET). Im Innern des ersten Kunststoffröhrchens 10 ist ein koaxiales und zylindrisches Innenrohr 30 angeordnet, welches aus dem Endbereich 11 des ersten Kunststoffröhrchens 10 heraus ragt. Der Aussendurchmesser 31 des Innenrohrs 30 entspricht dabei in etwa dem Innendurchmesser 13 des ersten Kunststoffröhrchens 10.

Über einen aus dem ersten Kunststoffröhrchen 10 herausragenden Bereich des Innenröhrchens 30 ist als rohrförmiges Verbindungsstück koaxial ein zylindrisches Polyimidröhrchen 50 geschoben. Der Innendurchmesser des Polyimidröhrchens 50 entspricht ungefähr dem Innendurchmesser 13 des ersten Kunststoffröhrchens 10. Im Bereich des ersten Endes 51 des Polyimidröhrchens 50, welches dem ersten Kunststoffröhrchen 10 zugewandt ist, steht ein erster Flansch 51.1 von der äusseren Mantelfläche des ersten Kunststoffröhrchens 10 ab. Der erste Flansch 51.1 weist einen halbkreisförmigen Querschnitt auf und umläuft das Polyimidröhrchen 50 vollständig, was in Fig. 1a jedoch nicht sichtbar ist. Im Bereich des zweiten Endes 52 des Polyimidröhrchens 50, steht ein zweiter Flansch 52.1 von der äusseren Mantelfläche des Polyimidröhrchen 50 ab. Der zweite Flansch 52.1 weist wie der erste Flansch 51.1 einen halbkreisförmigen Querschnitt auf und umläuft das Polyimidröhrchen 50 vollständig, was in Fig. 1a ebenfalls nicht sichtbar ist.

Fig. 1b zeigt die Situation nachdem das erste Ende 51 des Polyimidröhrchens 50 in das erste Kunststoffröhrchen 10 eingeschoben wurde und ein zweites Kunststoffröhrchen 20 auf das zweite Ende 52 des Polyimidröhrchens 50 aufgeschoben wurde.

Dabei liegt das erste Ende 51 des Polyimidröhrchens 50 im Endbereich 11 des ersten Kunststoffröhrchens 10 vor. Der Endbereich 11 des ersten Kunststoffröhrchens 10 umgibt also das erste Ende des Polyimidröhrchens 50 vollständig. In einer Längsrichtung des Polyimidröhrchens 50 überragt dabei der Endbereich 11 des ersten Kunststoffröhrchens 10 den ersten Flansch 51.1 des Polyimidröhrchens 50. Aufgrund des eingeschobenen Polyimidröhrchens 50 ist der Endbereich 11 des ersten Kunststoffröhrchen 10 in radialer Richtung leicht aufgeweitet.

Über das zweite Ende 52 des Polyimidröhrchens 50 ist des Weiteren ein Endbereich 21 eines zweiten Kunststoffröhrchens 20 geschoben. Das zweite Kunststoffröhrchen 20 ragt dabei koaxial in Längsrichtung über den zweiten Flansch 52 des Polyimidröhrchens 50. Das zweite Kunststoffröhrchen 20 ist ebenfalls zylindrisch, besteht z. B. aus Nylon und weist einen Innendurchmesser 23 von beispielsweise 0.7 mm bei einer Wandstärke 24 von z. B. 0.1 mm auf. Der Endbereich 21 des zweiten Kunststoffröhrchens 20 ist aufgrund des eingeschobenen Polyimidröhrchens 50 in radialer Richtung ebenfalls leicht aufgeweitet. Das erste Kunststoffröhrchen 10 und das zweite Kunststoffröhrchen 20 haben bezüglich ihren Durchmessern 13, 23 und Wandstärken 14, 24 demnach die gleichen Dimensionen und liegen sich mit ihren Stirnseiten koaxial gegenüber.

Ein Elastizitätsmodul des Polyimidröhrchens 50 ist dabei grösser als ein Elastizitätsmodul des ersten Kunststoffröhrchens 10, während ein Elastizitätsmodul des zweiten Kunststoffröhrchens 20 kleiner ist als das Elastizitätsmodul des ersten Kunststoffröhrchens 10.

Fig. 1c zeigt die Situation nachdem der Endbereich 11 des ersten Kunststoffröhrchens 10 in Längsrichtung über den Endbereich 21 des zweiten Kunststoffröhrchens 20 geschoben wurde. Das erste Kunststoffröhrchen 10 ragt dabei in Längsrichtung über das zweite Ende 52 des Polyimidröhrchens 50 und über den aufgeweiteten Endbereich 21 des zweiten Kunststoffröhrchens 20 hinaus. Der Endbereich 11 des ersten Kunststoffröhrchens 10 umgibt daher den Endbereich 21 des zweiten Kunststoffröhrchens 20 vollständig und ist im Überlappungsbereich in radialer Richtung teilweise zusätzlich aufgeweitet. Ein an den Endbereich 11 anschliessender hinterer Bereich 12 des ersten Kunststoffröhrchens 10 umgibt des Weiteren den nicht durch das zweite Kunststoffröhrchen 20 umgebenen Bereich des Polyimidröhrchens 50.

Rund um den Endbereich 11 und um den hinteren Bereich 12 des ersten Kunststoffröhrchens 10 ist ein Schrumpfschlauch 40 lose angeordnet. Der Schrumpfschlauch 40 umgibt die aufgeweiteten Bereiche der beiden Kunststoffröhrchen 10, 20 vollständig.

Die in Fig. 1c dargestellte Anordnung entspricht der Ausgangssituation direkt vor dem Umformprozess.

In Fig. 1d ist eine erste erfindungsgemässe Anordnung 1 aus den beiden Kunststoffröhrchen 10, 20 und dem Polyimidröhrchen 50 nach erfolgtem Umformprozess dargestellt. Der in Fig. 1c dargestellte Schrumpfschlauch 40 wurde nach dem Umformprozess wieder entfernt.

Aufgrund des Umformprozesses ist der Endbereich 21 des zweiten Kunststoffröhrchens 20 an den Bereich des zweiten Endes 52 des Polyimidröhrchens 50 angeformt und mit diesem adhesiv verbunden. Da der Endbereich 21 des zweiten Kunststoffröhrchens 20 auch an den zweiten Flansch 52.1 angeformt ist, liegt zudem eine formschlüssige Verbindung zwischen dem Endbereich 21 des zweiten Kunststoffröhrchens 20 und dem Polyimidröhrchen 50 vor.

Der Endbereich 11 des ersten Kunststoffröhrchens 10 ist von aussen an den Endbereich 21 des zweiten Kunststoffröhrchens 20 angeformt und mit diesem adhesiv verbunden. Der hintere Bereich 12 des ersten Kunststoffröhrchens 10 ist an das erste Ende 51 des Polyimidröhrchens 50 und damit auch an den ersten Flansch 51.1 angeformt, wodurch eine adhesive und formschlüssige Verbindung zwischen erstem Kunststoffröhrchen 10 und Polyimidröhrchen 50 vorliegt.

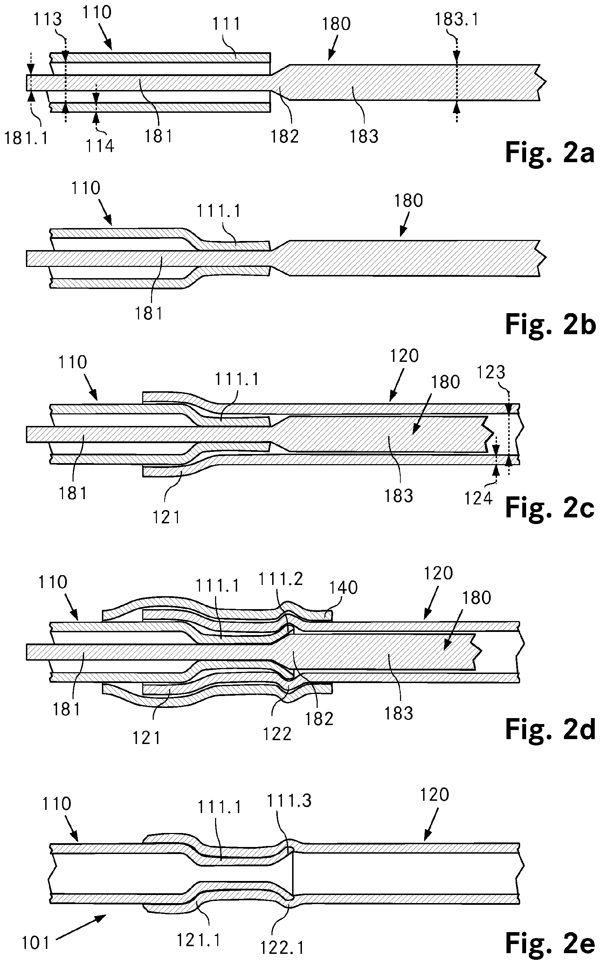

In den Fig. 2a - 2d sind verschiedene Zwischenstufen dargestellt, welche bei der Durchführung eines zweiten erfindungsgemässen Verfahrens durchlaufen werden. In Fig. 2e ist eine zweite durch das erfindungsgemässe Verfahren herstellbare Anordnung 2 abgebildet.

In Fig. 2a ist auf der linken Seite ein Endbereich 111 eines ersten zylindrischen Kunststoffröhrchens 110 im Längsschnitt abgebildet. Das erste Kunststoffröhrchen 110 weist einen Innendurchmesser 113 von beispielsweise 0.7 mm bei einer Wandstärke 114 von z. B. 0.1 mm auf und besteht aus Polyethylentherephtalat (PET). In das Innere des Endbereichs 111 ragt koaxial ein Endabschnitt 181 eines zylindrischen Drahtstücks 180. Der Aussendurchmesser 181.1 des Endabschnitts 181 des zylindrischen Drahtstücks 180 misst ca. 0.5 mm, was ca. 71% des Innendurchmessers 113 des ersten Kunststoffröhrchens 110 entspricht. Ausserhalb des ersten Kunststoffröhrchens 110 geht das zylindrische Drahtstück in einen sich konisch aufweitenden Übergangsbereich 182 über, an welchen wiederum ein verdickter Bereich 183 des zylindrischen Drahtstücks 180 anschliesst. Der Aussendurchmesser 183.1 des verdickten Bereichs des zylindrischen Drahtstücks 180 misst ca. 0.7 mm und entspricht damit im Wesentlichen dem Innendurchmesser 113 des ersten Kunststoffröhrchens 110.

Fig. 2b zeigt die Situation nachdem der Endbereich 111 des ersten Kunststoffröhrchens 110 an den Endabschnitt 181 des zylindrischen Drahtstücks 180 angeformt und dadurch zu einem Verbindungsstutzen 111.1 umgeformt wurde. Der Innendurchmesser des Verbindungsstutzens 111.1 entspricht damit dem Aussendurchmesser des Endabschnitts 181 des zylindrischen Drahtstücks 180 und weist im Vergleich zu den übrigen Bereichen des ersten Kunststoffröhrchens 110 einen verjüngten Aussendurchmesser sowie einen verjüngten Innendurchmesser auf.

Fig. 2c zeigt die Situation nachdem ein zweites Kunststoffröhrchen 120 koaxial über den verdickten Bereich 183 des zylindrischen Drahtstücks 180 und den Verbindungsstutzen 111.1 des ersten Kunststoffröhrchens 110 geschoben wurde. Das zweite Kunststoffröhrchen 120 ist ebenfalls zylindrisch, besteht z. B. aus Nylon und weist einen Innendurchmesser 123 von beispielsweise 0.7 mm bei einer Wandstärke 124 von z. B. 0.1 mm auf. Ein Endbereich 121 des zweiten Kunststoffröhrchens ragt dabei in Längsrichtung über den Verbindungsstutzen 111.1 des ersten Kunststoffröhrchens 110 hinaus und liegt hinter dem Verbindungsstutzen 111.1 auf einem nicht verjüngten Bereich des ersten Kunststoffröhrchens 110 auf. Der Endbereich 121 des ersten Kunststoffröhrchens 120 ist dabei in radialer Richtung leicht aufgeweitet.

Ein Elastizitätsmodul des zweiten Kunststoffröhrchens 120 ist dabei kleiner als ein Elastizitätsmodul des ersten Kunststoffröhrchens 110.

Fig. 2d zeigt die Situation nachdem das zylindrische Drahtstück 180 in Längsrichtung teilweise in den umgeformten Endbereich 111.1 des ersten Kunststoffröhrchens 110 hinein geschoben wurde. Der konische Übergangsbereich 182 des zylindrischen Drahtstücks 180 liegt dabei in einem Bereich eines freien Endes 111.2 des Verbindungsstutzens 111.1 vor. Dadurch liegt das freie Ende 111.2 des Verbindungsstutzens 111.1 im Wesentlichen konisch aufgeweitet und von Innen gegen das umgebende zweite Kunststoffröhrchen 120 gedrückt vor.

Im Bereich des aufgeweiteten freien Endes 111.2 des ersten Kunststoffröhrchens 110, welcher vom zweiten Kunststoffröhrchen 120 umgeben ist, weist das zweite Kunststoffröhrchen 120 einen ausgebuchteten Bereich 122 auf, in welchen das aufgeweitete Ende 111.2 des ersten Kunststoffröhrchens 110 eingreift.

Ausserhalb der beiden Kunststoffröhrchen 110, 120 ist ein Schrumpfschlauch 140 lose angeordnet, welcher einen Überlappungsbereich der beiden Kunststoffröhrchen vollständig umgibt.

In Fig. 2e ist eine zweite erfindungsgemässe Anordnung 101 aus den beiden Kunststoffröhrchen 110, 120 nach erfolgtem Umformprozess dargestellt. Der in Fig. 2c dargestellte Schrumpfschlauch 140 und das zylindrische Drahtstück wurden nach dem Umformprozess entfernt. Augrund des Umformprozesses liegt am Verbindungsstutzen 111.1 ein umgeformtes freies Ende 111.3 vor, welches formstabil ist.

Der Endbereich des zweiten Kunststoffröhrchens 120 liegt nun als umgeformter Endbereich 121.1 vor, welcher in einem vorderen Bereich von aussen an den Verbindungsstutzen 111.1 des ersten Kunststoffröhrchen 110 und hinter dem Verbindungsstutzen 111.1 an das erste Kunststoffröhrchen 110 angeformt ist. Weiter hinten weist das zweite Kunststoffröhrchen 120 einen umgeformten ausgebuchteten Bereich 122.1 auf, welcher von aussen an das aufgeweitete und umgeformte freie Ende 111.3 des ersten Kunststoffröhrchens 110 angeformt ist.

Damit liegt zwischen dem Verbindungsstutzen 111.1 des ersten Kunststoffröhrchens 110 und dem zweiten Kunststoffröhrchen eine adhesive und zugleich formschlüssige Verbindung vor, welche hohen Zugbelastungen standhält.

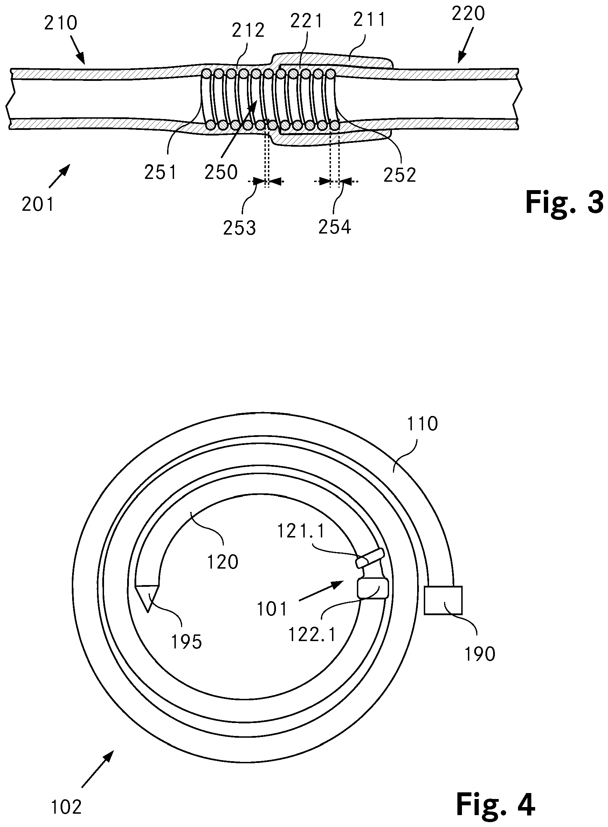

Die zweite Anordnung 102 ist z. B. Bestandteil eines medizinischen Katheters 102, welcher in Fig. 4 dargestellt ist.

Fig. 3 zeigt eine dritte erfindungsgemässe Anordnung 201, welche im Wesentlichen wie die erste Anordnung 1 hergestellt wurde und z. B. Bestandteil eines medizinischen Katheters ist. Anstelle des in der ersten Anordnung 1 vorliegenden Polyimidröhrchens 50, wurde bei der dritten Anordnung 201 jedoch eine Schraubenfeder 250 als rohrförmiges Verbindungsstück zur Verbindung eines ersten Kunststoffröhrchens 210 und eines zweiten Kunststoffröhrchens 220 verwendet.

Die Schraubenfeder 250 besteht beispielsweise aus rostfreiem Stahldraht mit einem Drahtdurchmesser 254 von z. B. 0.1 mm auf. Der Innendurchmesser der Schraubenfeder ist auf einer gesamten Länge konstant und misst beispielsweise ca. 0.7 mm, während die einzelnen Windungen der Schraubenfeder 250 einen Abstand 253 von z. B. 0.03 mm aufweisen. Insgesamt hat die Schraubenfeder 250 zehn Windungen.

Das erste Kunststoffröhrchen 210 ist zylindrisch und weisst einen Innendurchmesser von beispielsweise 0.7 mm bei einer Wandstärke von z. B. 0.1 mm auf und besteht aus Polyethylentherephtalat (PET). Das zweite Kunststoffröhrchen 20 ist ebenfalls zylindrisch, besteht z. B. aus Nylon und weisst einen Innendurchmesser 23 von beispielsweise 0.7 mm bei einer Wandstärke 24 von z.B. 0.1 mm auf. Ein Elastizitätsmodul des zweiten Kunststoffröhrchens 220 ist dabei kleiner ist als ein Elastizitätsmodul des ersten Kunststoffröhrchens 210.

Ein hinter einem Endbereich 211 des erstes Kunststoffröhrchen 210 liegender hinterer Bereich 212 des ersten Kunststoffröhrchens ist dabei koaxial von aussen an einen Bereich des ersten Endes 251 der Schraubenfeder 250 bzw. an sechs erste Windungen der Schraubenfeder 250 angeformt. Ein Endbereich 221 des zweiten Kunststoffröhrchens 220 ist ebenfalls koaxial von aussen an einen Bereich des zweiten Endes 252 der Schraubenfeder 250 bzw. an die vier verbleibenden Windungen der Schraubenfeder 250 angeformt. Aufgrund der Anformung ist die Schraubenfeder 250 sowohl im ersten Kunststoffröhrchen 210 und dem zweiten Kunststoffröhrchen 220 teilweise eingebettet, wobei die Schraubenfeder 250 bis ca. zu einer Tiefe eines halben Drahtdurchmessers 254 der Schraubenfeder 250 in den beiden Kunststoffröhrchen 210, 220 eingelassen ist. Damit besteht zwischen den beiden Kunststoffröhrchen 210, 220 und der Schraubenfeder 250 sowohl eine adhesive, wie auch eine formschlüssige Verbindung.

Der Endbereich 211 des ersten Kunststoffröhrchens 210, welcher stufenartig an den hinteren Bereich 212 des ersten Kunststoffröhrchens anschliesst, ist zudem von aussen an den Endbereich 221 des zweiten Kunststoffröhrchens 220 angeformt und mit diesem adhesiv verbunden.

Die Schraubenfeder 250 garantiert insbesondere bezüglich einer Längsrichtung der dritten erfindungsgemässen Anordnung 201 eine hohe Biegeflexibilität, gleichzeitig ist jedoch eine hohe Zugfestigkeit der dritten Anordnung 201 gegeben.

Die dritte Anordnung 102 ist daher insbesondere im Bereich der vordersten 30 cm eines Katheters geeignet, da dieser Bereich des Katheters eine hohe Biegeflexibilität aufweisen muss.

Fig. 4 zeigt einen medizinischen Katheter 102, welcher die zweite Anordnung 101 aus Fig. 2e beinhaltet. Auf der rechten Seite in Fig. 4 liegt ein Anschlussstutzen 190 des medizinischen Katheters 102 vor, welcher in das erste Kunststoffröhrchen 110 mündet. Die beiden verbundenen Kunststoffröhrchen 110, 120, welche die in Fig. 2e im Detail gezeigte zweite Anordnung 102 bilden, sind dabei in Form einer umgeformten und formstabilen Spirale vor, welche durch eine vorgängige Wärmebehandlung bei 50°C gebildet wurde. Am zweiten Kunststoffröhrchen 120 ist zudem in an sich bekannter Weise eine Katheterspitze 195 angeordnet.

Die in den Fig. 1c und 2d dargestellten Zwischenstufen werden einem Umformprozess unterworfen. Hierfür werden Schrumpfschläuche 40, 140 aus den Fig. 1c und 2d beispielsweise mit Heissluft angeblasen. Wichtig dabei ist, dass die umzuformenden Kunststoffröhrchen ausreichend erwärmt werden, so dass wenigstens ein Anschmelzen der Kunststoffröhrchen eintritt. Die hierfür erforderlichen Prozessparameter wie Temperatur und Dauer des Anblasens hängen einerseits vom Material der verwendeten Kunststoffröhrchen und andererseits von der verwendeten Heissluftquelle ab. Optimale Prozessparameter können z. B. im Rahmen von Testversuchen in einfacher Weise ermittelt werden.

Die vorangehenden Ausführungsformen sind lediglich als illustrative Beispiele zu verstehen, welche im Rahmen der Erdung beliebig abgewandelt werden können.

So kann anstelle eines Polyimidröhrchens 50 mit Flanschen 51, 52 auch ein an der Aussenseite unstrukturiertes Polyimidröhrchen ohne Flansche eingesetzt werden. In diesem Fall wird im Wesentlichen eine adhesive Verbindung zwischen Kunststoffröhrchen und Polyimidröhrchen gebildet. Auch möglich ist die Verwendung eines Polyimidröhrchens, welches über ein Aussengewinde und/oder Nuten verfügt oder die Verwendung eines Röhrchens aus einem anderen Material, welches auch bei höheren Temperaturen eine hohe Festigkeit und Formstabilität aufweist.

Ebenso ist es möglich, anstelle des Polyimidröhrchens ein Röhrchen, beispielsweise aus einem Kunststoff zu verwenden, welches mit einer Drahtwendel und/oder Fasermaterialien verstärkt ist.

Auch ist es grundsätzlich denkbar, auf das Anbringen von Schrumpfschläuchen 40, 140 zu verzichten und stattdessen den Umformprozess beispielsweise mit einer Pressmaschine durchzuführen.

Es ist auch denkbar, dass das rohrförmige Verbindungsstück bzw. das Polyimidröhrchen 50 und/oder die Schraubenfeder 250 Bereiche mit unterschiedlichen Innen- und/oder Aussendurchmessern aufweisen.

Dies kann insbesondere zweckmässig sein, wenn Kunststoffröhrchen mit unterschiedlichen Innen- und/oder Aussendurchmessern mit dem erfindungsgemässen Verfahren miteinander zu verbinden sind.

Das in den Fig. 1a - 1d illustrierte Verfahren kann zudem auch ohne ein Innenrohr 30 durchgeführt werden. Der Innendurchmesser des Polyimidröhrchens 50 kann in diesem Fall auch geringer oder grösser sein, als der Innendurchmesser der Kunststoffröhrchen. Ebenso kann die Schraubenfeder 250 aus Fig. 3 einen geringeren oder grösseren Innendurchmesser aufweisen als die Kunststoffröhrchen.

Das zylindrische Drahtstück 180 aus Fig. 2a kann z.B. anstelle des sich konisch aufweitenden Übergangsbereichs 182 auch einen anders ausgebildeten Übergangsbereich aufweisen, welcher z. B. im Wesentlichen stufenartig ausgebildet ist. Auch denkbar ist die Verwendung eines zylindrischen Drahtstücks, dessen Endabschnitt vollständig konisch ausgebildet ist.

Bei dem in Fig. 4 abgebildeten medizinischen Katheter 102 ist es auch möglich, dass mehr als zwei miteinander verbundene Kunststoffröhrchen vorliegen. Anstelle oder zusätzlich zur Anordnung 101 kann der medizinische Katheter 102 z. B. auch weitere Verbindungen zwischen Kunststoffröhrchen enthalten. Dies können z. B. durch die in den Fig. 1a - 1d und in den Fig. 2a - 2e veranschaulichten Verfahren hergestellt werden. Insbesondere im Bereich der Spitze ist dabei die Verbindung der Kunststoffröhrchen durch eine Schraubenfeder, wie z. B. in Fig. 3 dargestellt, vorteilhaft, da sich diese Art im Besonderen durch eine hohe Biegeflexibilität auszeichnet.

Grundsätzlich ist es mit den erfindungsgemässen Verfahren auch möglich, Ballone, welche z. B. als rohrförmige Elemente aus Kunststoff vorliegen können, an einem Katheterschaft zu befestigen. Damit eignet sich das erfindungsgemässe Verfahren auch bei der Herstellung von Ballonkathetern.

Zusammenfassend ist festzustellen, dass ein besonders flexibles Verfahren geschaffen wurde, welches eine sichere und qualitativ hochstehende Verbindung von dünnen Kunststoffröhrchen mit äusserst geringen Dimensionen aus unterschiedlichsten Materialien ermöglicht. Dabei kann insbesondere auf Klebstoffe und die bezüglich Materialauswahl limitierten Schweissprozesse verzichtet werden. Die erfindungsgemäss hergestellten Anordnungen bzw. Verbindungen aus den beiden Kunststoffröhrchen weisen eine äusserst hohe mechanische Festigkeit auf, so dass diese insbesondere auch hohen Zugbelastungen standhalten. Besonders vorteilhaft ist das erfindungsgemässe Verfahren bei der Herstellung von Kathetern anwendbar.

## Patentansprüche

1. Verfahren zum koaxialen Verbinden eines ersten Kunststoffröhrchens (10, 110) mit einem zweiten Kunststoffröhrchen (20, 120), welche Kunststoffröhrchen (10, 20) insbesondere als Bestandteile eines medizinischen Katheters (102) vorgesehen sind, wobei die beiden Kunststoffröhrchen (10, 20) über ein rohrförmiges Verbindungsstück (50) verbunden werden, **dadurch gekennzeichnet, dass** das erste Kunststoffröhrchen (10, 110) und das zweite Kunststoffröhrchen (20, 120) in einem Umformprozess von aussen an das rohrförmige Verbindungsstück (50), dessen Wandung mit einer eingebetteten Drahtwendel verstärkt ist, angeformt werden, so dass eine adhesive oder formschlüssige Verbindung ausgebildet wird.

2. Verfahren nach Anspruch 1, **dadurch gekennzeichnet, dass** das erste Kunststoffröhrchen (10) und/oder das zweite Kunststoffröhrchen (20) während dem Umformprozess durch eine Wärmebehandlung erweicht wird und durch eine in einer radialen Richtung einwirkende Presskraft an das rohrförmige Verbindungsstück (50) angeformt wird.

3. Verfahren nach einem der Ansprüche 1 - 2, **dadurch gekennzeichnet, dass** vor dem Umformprozess ein Endbereich (11) des ersten Kunststoffröhrchens (10) koaxial über ein erstes Ende (51) des rohrförmigen Verbindungsstücks (50) geschoben wird und ein Endbereich (21) des zweiten Kunststoffröhrchens (20) koaxial über ein zweites Ende (52) des rohrförmigen Verbindungsstücks (50) geschoben wird.

4. Verfahren nach Anspruch 3, **dadurch gekennzeichnet, dass** der Endbereich (11) des ersten Kunststoffröhrchens (10) vor dem Umformprozess zusätzlich koaxial über den Endbereich (21) das zweiten Kunststoffröhrchen (20) geschoben wird.

5. Verfahren nach einem der Ansprüche 1 - 2, **dadurch gekennzeichnet, dass** vor dem Umformprozess der Endbereich (111) des ersten Kunststoffröhrchens (110) zu einem Verbindungsstutzen (111.1) umgeformt wird, wobei der Verbindungsstutzen (111.1) als rohrförmiges Verbindungsstück vorgesehen ist.

6. Verfahren nach Anspruch 5, **dadurch gekennzeichnet, dass** ein Aussendurchmesser des Verbindungsstutzens (111.1) verglichen mit einem Aussendurchmesser des ersten Kunststoffröhrchens (110) im Wesentlichen verjüngt ausgebildet wird.

7. Verfahren nach einem der Ansprüche 5 - 6, **dadurch gekennzeichnet, dass** zur Herstellung des Verbindungsstutzens (111.1) ein Endabschnitt (181) eines zylindrischen Drahtstücks (180) in den Endbereich (111) des ersten Kunststoffröhrchens (110) eingeführt wird und anschliessend der Endbereich (111) des ersten Kunststoffröhrchens (110) durch Umformung von aussen an den Endabschnitt (181) des zylindrischen Drahtstücks (180) angeformt wird, wobei ein Aussendurchmesser (181.1) des Endabschnitts (181) des zylindrischen Drahtstücks (180) insbesondere 60 - 80%, besonders bevorzugt 65 - 75%, des Innendurchmessers (113) des ersten Kunststoffröhrchens (111) vor der Ausbildung des Verbindungsstutzens (111.1) misst.

8. Verfahren nach einem der Ansprüche 5 - 7, **dadurch gekennzeichnet, dass** das zweite Kunststoffröhrchen (120) vor dem Umformprozess koaxial und wenigstens über eine gesamte Länge des Verbindungsstutzens (111.1) über diesen geschoben wird.

9. Verfahren nach einem der Ansprüche 5 - 8, **dadurch gekennzeichnet, dass** ein freies Ende (111.2) des Verbindungsstutzens (111.2) vor dem Umformprozess aufgeweitet wird.

10. Verfahren nach Anspruch 9, **dadurch gekennzeichnet, dass** zur Aufweitung des freien Endes (111.2) des Verbindungsstutzens (111.1) ein an den Endabschnitt (181) des zylindrischen Drahtstücks (180) anschliessender verdickter Bereich (183) und/oder ein aufgeweiteter Übergangsbereich (182) zum verdickten Bereich (183) des zylindrischen Drahtstücks (180) in einer Richtung zum ersten Kunststoffröhrchen (110) hin in das freie Ende (111.2) des Verbindungsstutzens (111.1) hinein geschoben wird, wobei bevorzugt ein Aussendurchmesser des verdickten Bereichs (183) des zylindrischen Drahtstücks (180) im Wesentlichen dem Innendurchmesser (113) des ersten Kunststoffröhrchens (110) vor der Herstellung des Verbindungsstutzens (111.1) entspricht.

11. Verfahren nach einem der Ansprüche 9 - 10, **dadurch gekennzeichnet, dass** während dem Umformprozess das zweite Kunststoffröhrchen (120) unter Ausbildung einer formschlüssigen Verbindung an den endseitig aufgeweiteten Verbindungsstutzen (111.1) angeformt wird.

12. Verfahren nach einem der Ansprüche 10 - 11, **dadurch gekennzeichnet, dass** während dem Umformprozess der Endabschnitt (181) des zylindrischen Drahtstücks (180) im Verbindungsstutzen (111.1) und der aufgeweitete Übergangsbereich (182) und/oder der verdickte Bereich (183) des zylindrischen Drahtstücks (180) im aufgeweiteten freien Ende (111.2) des Verbindungsstutzens (111.2) verbleiben.

13. Verfahren nach einem der Ansprüche 1 - 12, **dadurch gekennzeichnet, dass** das erste Kunststoffröhrchen (110) und/oder das zweite Kunststoffröhrchen (120) nach dem Umformprozess spiralförmig aufgewickelt werden und unter Anwendung von Wärme zu einer formstabilen Spirale geformt werden.

14. Anordnung (1) umfassend ein erstes Kunststoffröhrchen (10, 110), welches über ein rohrförmiges Verbindungsstück (50) mit einem zweiten Kunststoffröhrchen (10, 110) verbunden ist, insbesondere hergestellt durch ein Verfahren nach einem der Ansprüche 1 - 13, wobei das erste Kunststoffröhrchen (10, 110) und das zweite Kunststoffröhrchen (20, 120) von aussen an das rohrförmige Verbindungsstück (50) angeformt und adhesiv oder formschlüssig mit dem Verbindungsstück (50) verbunden ist, **dadurch gekennzeichnet, dass** eine Wandung des rohrförmigen Verbindungsstücks (50) mit einer eingebetteten Drahtwendel verstärkt ist.

15. Anordnung nach Anspruch 14, **dadurch gekennzeichnet, dass** das erste Kunststoffröhrchen (10, 110) aus einem ersten thermoplastischen Material, insbesondere aus Polyethylentherephtalat, besteht und dass das zweite Kunststoffröhrchen (20, 120) aus einem zweiten thermoplastischen Material, insbesondere Nylon, besteht.

16. Anordnung nach einem der Ansprüche 14 - 15, **dadurch gekennzeichnet, dass** ein Elastizitätsmodul des ersten Kunststoffröhrchens (10, 110) grösser ist als ein Elastizitätsmodul des zweiten Kunststoffröhrchens (20, 120).

17. Anordnung nach Anspruch 16, **dadurch gekennzeichnet, dass** ein Elastizitätsmodul des rohrförmigen Verbindungsstücks (50) grösser ist als das Elastizitätsmodul des ersten Kunststoffröhrchens (10, 110) und grösser als das Elastizitätsmodul des zweiten Kunststoffröhrchens (20, 120) und dass insbesondere das rohrförmige Verbindungsstück (50) aus Polyimid besteht.

18. Anordnung nach einem der Ansprüche 14 - 17, **dadurch gekennzeichnet, dass** das rohrförmige Verbindungsstück (50) eine strukturierte äussere Mantelfläche aufweist und dass die strukturierte äussere Mantelfläche insbesondere Vorstände und/oder Rillen und/oder Schraubengewinde und/oder umlaufende Flansche (51.1, 51.2) umfasst.

19. Anordnung nach einem der Ansprüche 14 - 18, **dadurch gekennzeichnet, dass** das erste Kunststoffröhrchen (110) und/oder das zweite Kunststoffröhrchen (120) in Form einer formstabilen Spirale vorliegen.

20. Katheter (102) für medizinische Zwecke, umfassend eine Anordnung (101) nach einem der Ansprüche 14 - 19.

21. Verwendung eines Verfahrens nach einem der Ansprüche 1 - 13 zur Herstellung eines Katheters (102).

## Claims

1. Method for coaxially connecting a first plastic tube (10, 110) to a second plastic tube (20, 120), which plastic tubes (10, 20) are provided in particular as component parts of a medical catheter (102), the two plastic tubes (10, 20) being connected by way of a tubular connecting piece (50), **characterized in that** the first plastic tube (10, 110) and the second plastic tube (20, 120) are integrally molded from the outside onto the tubular connecting piece (50), the wall of which is reinforced with an embedded wire coil, in a forming process, so that an adhesive or positive connection is formed.

2. Method according to Claim 1, **characterized in that** the first plastic tube (10) and/or the second plastic tube (20) are softened during the forming process by a heat treatment and are integrally molded onto the tubular connecting piece (50) by a pressing force acting in a radial direction.

3. Method according to either of Claims 1 and 2, **characterized in that**, before the forming process, an end region (11) of the first plastic tube (10) is pushed coaxially over a first end (51) of the tubular connecting piece (50) and an end region (21) of the second plastic tube (20) is pushed coaxially over a second end (52) of the tubular connecting piece (50).

4. Method according to Claim 3, **characterized in that** the end region (11) of the first plastic tube (10) is additionally pushed coaxially over the end region (21) of the second plastic tube (20) before the forming process.

5. Method according to either of Claims 1 and 2, **characterized in that**, before the forming process, the end region (111) of the first plastic tube (110) is formed into a connecting stub (111.1), the connecting stub (111.1) being provided as a tubular connecting piece.

6. Method according to Claim 5, **characterized in that** an outside diameter of the connecting stub (111.1) is formed such that it is substantially narrowed in comparison with an outside diameter of the first plastic tube (110).

7. Method according to either of Claims 5 and 6, **characterized in that**, to produce the connecting stub (111.1), an end portion (181) of a cylindrical piece of wire (180) is inserted into the end region (111) of the first plastic tube (110) and subsequently the end region (111) of the first plastic tube (110) is integrally molded from the outside onto the end portion (181) of the cylindrical piece of wire (180) by forming, an outside diameter (181.1) of the end portion (181) of the cylindrical piece of wire (180) measuring particularly 60 - 80%, particularly preferably 65 - 75%, of the inside diameter (113) of the first plastic tube (111) before the formation of the connecting stub (111.1).

8. Method according to one of Claims 5 to 7, **characterized in that**, before the forming process, the second plastic tube (120) is pushed coaxially over the connecting stub (111.1), at least over the entire length thereof.

9. Method according to one of Claims 5 to 8, **characterized in that** a free end (111.2) of the connecting stub (111.2) is widened before the forming process.

10. Method according to Claim 9, **characterized in that**, to widen the free end (111.2) of the connecting stub (111.1), a thickened region (183) that adjoins the end portion (181) of the cylindrical piece of wire (180) and/or a widened transitional region (182) with respect to the thickened region (183) of the cylindrical piece of wire (180) is pushed into the free end (111.2) of the connecting stub (111.1) in a direction toward the first plastic tube (110), an outside diameter of the thickened region (183) of the cylindrical piece of wire (180) preferably corresponding substantially to the inside diameter (113) of the first plastic tube (110) before the production of the connecting stub (111.1).

11. Method according to either of Claims 9 and 10, **characterized in that**, during the forming process, the second plastic tube (120) is integrally molded onto the connecting stub (111.1) widened at the end, thereby forming a positive connection.

12. Method according to either of Claims 10 and 11, **characterized in that**, during the forming process, the end portion (181) of the cylindrical piece of wire (180) remains in the connecting stub (111.1) and the widened transitional region (182) and/or the thickened region (183) of the cylindrical piece of wire (180) remains in the widened free end (111.2) of the connecting stub (111.2).

13. Method according to one of Claims 1 to 12, **characterized in that** the first plastic tube (110) and/or the second plastic tube (120) are spirally wound up after the forming process and formed into a dimensionally stable spiral under the application of heat.

14. Arrangement (1) comprising a first plastic tube (10, 110), which is connected to a second plastic tube (10, 110) by way of a tubular connecting piece (50), in particular produced by a method according to one of Claims 1 to 13, wherein the first plastic tube (10, 110) and the second plastic tube (20, 120) is integrally molded from the outside onto the tubular connecting piece (50) and adhesively or positively connected to the connecting piece (50), **characterized in that** a wall of the tubular connecting piece (50) is reinforced with an embedded wire coil.

15. Arrangement according to Claim 14, **characterized in that** the first plastic tube (10, 110) consists of a first thermoplastic material, in particular of polyethylene terephthalate, and **in that** the second plastic tube (20, 120) consists of a second thermoplastic material, in particular nylon.

16. Arrangement according to either of Claims 14 and 15, **characterized in that** a modulus of elasticity of the first plastic tube (10, 110) is greater than a modulus of elasticity of the second plastic tube (20, 120).

17. Arrangement according to Claim 16, **characterized in that** a modulus of elasticity of the tubular connecting piece (50) is greater than the modulus of elasticity of the first plastic tube (10, 110) and greater than the modulus of elasticity of the second plastic tube (20, 120) and **in that** in particular the tubular connecting piece (50) consists of polyimide.

18. Arrangement according to one of Claims 14 to 17, **characterized in that** the tubular connecting piece (50) has a structured outer lateral surface and **in that** the structured outer lateral surface comprises in particular projections and/or grooves and/or screw threads and/or peripheral flanges (51.1, 51.2).

19. Arrangement according to one of Claims 14 to 18, **characterized in that** the first plastic tube (110) and/or the second plastic tube (120) are in the form of a dimensionally stable spiral.

20. Catheter (102) for medical purposes, comprising an arrangement (101) according to one of Claims 14 to 19.

21. Use of a method according to one of Claims 1 to 13 for producing a catheter (102).

## Revendications

1. Procédé d'assemblage coaxial d'un premier tube capillaire en matière plastique (10, 110) avec un deuxième tube capillaire en matière plastique (20, 120), lesdits tubes capillaires en matière plastique (10, 20) étant notamment conçus comme des éléments constitutifs d'un cathéter médical (102), les deux tubes capillaires en matière plastique (10, 20) étant reliés par le biais d'une pièce d'assemblage (50) tubulaire, **caractérisé en ce que** le premier tube capillaire en matière plastique (10, 110) et le deuxième tube capillaire en matière plastique (20, 120), au cours d'un processus de façonnage, sont surmoulés depuis l'extérieur contre la pièce d'assemblage (50) tubulaire dont la paroi est renforcée avec un filament métallique spiralé, de sorte qu'un assemblage adhésif ou par complémentarité de formes est formé.

2. Procédé selon la revendication 1, **caractérisé en ce que** le premier tube capillaire en matière plastique (10) et/ou le deuxième tube capillaire en matière plastique (20) est ramolli par un traitement thermique durant le processus de façonnage puis surmoulé contre la pièce d'assemblage (50) tubulaire par une force de pressage agissant dans une direction radiale.

3. Procédé selon l'une des revendications 1 à 2, **caractérisé en ce qu'**avant le processus de façonnage, une zone d'extrémité (11) du premier tube capillaire en matière plastique (10) est glissée de manière coaxiale sur une première extrémité (51) de la pièce d'assemblage (50) tubulaire et une zone d'extrémité (21) du deuxième tube capillaire en matière plastique (20) est glissée de manière coaxiale sur une deuxième extrémité (52) de la pièce d'assemblage (50) tubulaire.

4. Procédé selon la revendication 3, **caractérisé en ce que** la zone d'extrémité (11) du premier tube capillaire en matière plastique (10) est en plus glissée de manière coaxiale sur la zone d'extrémité (21) du deuxième tube capillaire en matière plastique (20) avant le processus de façonnage.

5. Procédé selon l'une des revendications 1 à 2, **caractérisé en ce qu'**avant le processus de façonnage, la zone d'extrémité (111) du premier tube capillaire en matière plastique (110) est façonnée en un manchon d'assemblage (111.1), le manchon d'assemblage (111.1) étant conçu sous la forme d'une pièce d'assemblage tubulaire.

6. Procédé selon la revendication 5, **caractérisé en ce qu'**un diamètre extérieur du manchon d'assemblage (111.1), comparé à un diamètre extérieur du premier tube capillaire en matière plastique (110), est réalisé nettement rétrécit.

7. Procédé selon l'une des revendications 5 à 6, **caractérisé en ce que** pour la fabrication du manchon d'assemblage (111.1), une portion d'extrémité (181) d'un fil métallique cylindrique (180) est introduite dans la zone d'extrémité (111) du premier tube capillaire en matière plastique (110) et ensuite la zone d'extrémité (111) du premier tube capillaire en matière plastique (110) est surmoulée contre la portion d'extrémité (181) du fil métallique cylindrique (180) par façonnage depuis l'extérieur, le diamètre extérieur (181.1) de la portion d'extrémité (181) du fil métallique cylindrique (180) mesurant notamment de 60 à 80 %, notamment de préférence de 65 à 75 % du diamètre intérieur (113) du premier tube capillaire en matière plastique (110) avant la formation du manchon d'assemblage (111.1).

8. Procédé selon l'une des revendications 5 à 7, **caractérisé en ce qu'**avant le processus de façonnage, le deuxième tube capillaire en matière plastique (120) est glissé de manière coaxiale sur le manchon d'assemblage (111.1) et au moins sur une longueur totale de celui-ci.

9. Procédé selon l'une des revendications 5 à 8, **caractérisé en ce que** l'extrémité libre (111.2) du manchon d'assemblage (111.2) est élargie avant le processus de façonnage.

10. Procédé selon la revendication 9, **caractérisé en ce que** pour l'élargissement de l'extrémité libre (111.2) du manchon d'assemblage (111.1), une zone épaissie (183) rattachée à la portion d'extrémité (181) du fil métallique cylindrique (180) et/ou une zone de transition (182) élargie vers la zone épaissie (183) du fil métallique cylindrique (180) est glissée à l'intérieur dans l'extrémité libre (111.2) du manchon d'assemblage (111.1) dans une direction vers le premier tube capillaire en matière plastique (110), un diamètre extérieur de la zone épaissie (183) du fil métallique cylindrique (180) correspondant pour l'essentiel au diamètre intérieur (113) du premier tube capillaire en matière plastique (110) avant la fabrication du manchon d'assemblage (111.1).

11. Procédé selon l'une des revendications 9 à 10, **caractérisé en ce que** pendant le processus de façonnage, le deuxième tube capillaire en matière plastique (120) est surmoulé contre le manchon d'assemblage (111.1) élargi du côté de l'extrémité en formant un assemblage par complémentarité de formes.

12. Procédé selon l'une des revendications 10 à 11, **caractérisé en ce que** pendant le processus de façonnage, la portion d'extrémité (181) d'un fil métallique cylindrique (180) demeure dans le manchon d'assemblage (111.1) et la zone de transition (182) élargie et/ou la zone épaissie (183) du fil métallique cylindrique (180) dans l'extrémité libre (111.2) élargie du manchon d'assemblage (111.2).

13. Procédé selon l'une des revendications 1 à 12, **caractérisé en ce que** le premier tube capillaire en matière plastique (110) et/ou le deuxième tube capillaire en matière plastique (120) sont enroulés en forme de spirale après le processus de façonnage et une spirale indéformable est façonnée en utilisant de la chaleur.

14. Arrangement (1) comprenant un premier tube capillaire en matière plastique (10, 110), lequel est assemblé avec un deuxième tube capillaire en matière plastique (10, 110) par le biais d'une pièce d'assemblage (50) tubulaire, notamment fabrique par un procédé selon l'une des revendications 1 à 13, le premier tube capillaire en matière plastique (10, 110) et le deuxième tube capillaire en matière plastique (20, 120) étant surmoulés depuis l'extérieur contre la pièce d'assemblage (50) tubulaire et étant assemblé avec la pièce d'assemblage (50) tubulaire de manière adhésive ou par complémentarité de formes, **caractérisé en ce qu'**une paroi de la pièce d'assemblage (50) est renforcée avec un filament métallique spiralé enrobé.

15. Arrangement selon la revendication 14, **caractérisé en ce que** le premier tube capillaire en matière plastique (10, 110) se compose d'un premier matériau thermoplastique, notamment de polytéréphtalate d'éthylène, et **en ce que** le deuxième tube capillaire en matière plastique (20, 120) se compose d'un deuxième matériau thermoplastique, notamment de nylon.

16. Arrangement selon l'une des revendications 14 à 15, **caractérisé en ce qu'**un module d'élasticité du premier tube capillaire en matière plastique (10, 110) est supérieur à un module d'élasticité du deuxième tube capillaire en matière plastique (20, 120).

17. Arrangement selon la revendication 16, **caractérisé en ce qu'**un module d'élasticité de la pièce d'assemblage (50) tubulaire est supérieur au module d'élasticité du premier tube capillaire en matière plastique (10, 110) et supérieur au module d'élasticité du deuxième tube capillaire en matière plastique (20, 120) et notamment **en ce que** la pièce d'assemblage (50) tubulaire se compose de polyimide.

18. Arrangement selon l'une des revendications 14 à 17, **caractérisé en ce que** la pièce d'assemblage (50) tubulaire possède une enveloppe extérieure structurée et **en ce que** l'enveloppe extérieure structurée comporte notamment des parties saillantes et/ou des cannelures et/ou un filetage et/ou une bride périphérique (51.1, 51.2).

19. Arrangement selon l'une des revendications 14 à 18, **caractérisé en ce que** le premier tube capillaire en matière plastique (110) et/ou le deuxième tube capillaire en matière plastique (120) se présentent sous la forme d'une spirale indéformable.

20. Cathéter (102) à usage médical, comprenant un arrangement selon l'une des revendications 14 à 19.

21. Utilisation d'un procédé selon l'une des revendications 1 à 13 pour la fabrication d'un cathéter (102) .
